# EUROPEAN PATENT SPECIFICATION

(11) **EP 1 267 909 B1**
(45) Date of publication and mention of the grant of the patent: **28.07.2010**
(21) Application number: 01933358.2
(22) Date of filing: 29.03.2001
(51) Int. Cl.: A61K 38/17, A61K 39/00, A61K 45/00, C07K 4/12, C07K 14/705, C07K 16/28

(54) **METHODS OF BLOCKING TISSUE DESTRUCTION BY AUTOREACTIVE T CELLS**
VERFAHREN ZUR BLOCKIERUNG DER ZERSTÖRUNG VON GEWEBE MITTELS AUTOREAKTIVER T-ZELLEN
PROCEDES PERMETTANT D'INHIBER LA DESTRUCTION TISSULAIRE INDUITE PAR LES LYMPHOCYTES T AUTOREACTIFS

(30) Priority: 29.03.2000 US 192814 P
(43) Date of publication of application: 02.01.2003
(73) Proprietor: THE OHIO STATE UNIVERSITY RESEARCH FOUNDATION, Columbus, Ohio 43210-1063 (US)
(72) Inventor: LIU, Yang, Columbus, OH 43220 (US); ZHENG, Pan, Columbus, OH 43220 (US); BAI, Xue-Feng, Columbus, OH 43221 (US)
(74) Representative: Denison, Christopher Marcus
(86) International application number: PCT/US2001/040390
(87) International publication number: WO 2001/072325

(56) References cited:
- CHEN Y C ET AL: "Establishment and characterization of cloned CD4- CD8- alphabeta-T cell receptor (TCR)-bearing autoreactive T cells from autoimmune NZB x NZW F1 mice." CLINICAL AND EXPERIMENTAL IMMUNOLOGY. APR 1997, vol. 108, no. 1, April 1997 (1997-04), pages 52-57, XP002370649 ISSN: 0009-9104
- AIGNER S ET AL: "Heat stable antigen (mouse CD24) supports myeloid cell binding to endothelial and platelet P-selectin." INTERNATIONAL IMMUNOLOGY. OCT 1995, vol. 7, no. 10, October 1995 (1995-10), pages 1557-1565, XP009062524 ISSN: 0953-8178
- ENK A H ET AL: "Heat-stable antigen is an important costimulatory molecule on epidermal Langerhans' cells." JOURNAL OF IMMUNOLOGY (BALTIMORE, MD. : 1950) 1 APR 1994, vol. 152, no. 7, 1 April 1994 (1994-04-01), pages 3264-3270, XP002369718 ISSN: 0022-1767
- WENGER R H ET AL: "B-cell maturation in chimaeric mice deficient for the heat stable antigen (HSA/mouse CD24)." TRANSGENIC RESEARCH. MAY 1995, vol. 4, no. 3, May 1995 (1995-05), pages 173-183, XP009062537 ISSN: 0962-8819
- ERDMANN G ET AL: "Heat-stable antigen is expressed by murine keratinocytes and delivers costimulatory signals in T-cell activation." EXPERIMENTAL DERMATOLOGY. OCT 1995, vol. 4, no. 5, October 1995 (1995-10), pages 291-296, XP009062536 ISSN: 0906-6705
- HUBBE M ET AL: "HEAT-STABLE ANTIGEN/CD24 ON MOUSE T LYMPHOCYTES: EVIDENCE FOR A COSTIMULATORY FUNCTION" EUROPEAN JOURNAL OF IMMUNOLOGY, WEINHEIM, DE, vol. 24, no. 3, 1 March 1994 (1994-03-01), pages 731-737, XP000572870 ISSN: 0014-2980
- NIELSEN P J ET AL: "Altered erythrocytes and a leaky block in B-cell development in CD24/HSA-deficient mice." BLOOD. 1 FEB 1997, vol. 89, no. 3, 1 February 1997 (1997-02-01), pages 1058-1067, XP002369722 ISSN: 0006-4971
- PAWLIUK R ET AL: "Selection of retrovirally transduced hematopoietic cells using CD24 as a marker of gene transfer." BLOOD. 1 NOV 1994, vol. 84, no. 9, 1 November 1994 (1994-11-01), pages 2868-2877, XP008002532 ISSN: 0006-4971
- HAHNE M ET AL: "The heat-stable antigen can alter very late antigen 4-mediated adhesion." THE JOURNAL OF EXPERIMENTAL MEDICINE. 1 APR 1994, vol. 179, no. 4, 1 April 1994 (1994-04-01), pages 1391-1395, XP002369723 ISSN: 0022-1007
- STINISSEN P ET AL: "AUTOIMMUNE PATHOGENESIS OF MULTIPLE SCLEROSIS: ROLE OF AUTOREACTIVE T LYMPHOCYTES AND NEW IMMUNOTHERAPEUTIC STRATEGIES" CRITICAL REVIEWS IN IMMUNOLOGY, CRC PRESS, INC, vol. 17, no. 1, 1997, pages 33-75, XP009045645 ISSN: 1040-8401
- NIELSEN P J ET AL: "Constitutive expression of transgenic heat stable antigen (mCD24) in lymphocytes can augment a secondary antibody response." INTERNATIONAL IMMUNOLOGY. NOV 1993, vol. 5, no. 11, November 1993 (1993-11), pages 1355-1364, XP009062540 ISSN: 0953-8178
- BAI XUE-FENG ET AL: "CD24 controls expansion and persistence of autoreactive T cells in the central nervous system during experimental autoimmune encephalomyelitis." THE JOURNAL OF EXPERIMENTAL MEDICINE. 16 AUG 2004, vol. 200, no. 4, 16 August 2004 (2004-08-16), pages 447-458, XP002369725 ISSN: 0022-1007
- GUPTA A.: 'Sequence and structural homology between a mouse T complex protein TCP-1 and teh chaperonin family of bacterial GROEL 60-65-KDa heat shock antigen and eukaryotic proteins' BIOCHEM. INT. vol. 20, no. 4, April 1990, pages 833 - 841, XP002958334
- BAI X.F. ET AL.: 'The heat-stable antigen determines pathogenicity of self-reactive T cells in experimental autoimmune encephalomyelitis' J. CLIN. INVEST. vol. 105, no. 9, May 2000, pages 1227 - 1232, XP002944025
- WITHER J.E. ET AL.: 'Genetic dissection of B cell traits in New Zealand black mice. The expanded population of B cells expressing up-regulated costimulatory molecules shows linkage to Nba2' EUR. J. IMMUNOL. vol. 30, no. 2, February 2000, pages 356 - 365, XP002944026

## Description

### Background of the Invention

The present invention relates to agents and means for blocking deleterious T cell mediated immune reactions. Such reactions occur in autoimmune diseases, such as for example, multiple sclerosis (MS), rheumatoid arthritis, systemic lupus erythematosis, psoriasis, diabetes, and allergies. Such reactions also occur during rejection of transplants.

Two types of signals are required for T cell activation and proliferation. The first, which gives specificity to the immune response, involves an interaction between the T-cell receptor/CD3 complex and an antigenic peptide presented by major histocompatibility complex (MHC) class I or class II proteins on the surface of an antigen-presenting cell (APC). The second type of signal, called a costimulatory signal, involves interaction between receptor-ligand pairs expressed on the surface of APCs and T cells. One example of a costimulatory receptor-ligand pair is the B7 ligand and its counterpart receptor CD28. Engagement of CD28 by its ligands B7-1 or B7-2 on the surface of APCs initiates a signaling cascade culminating in cytokine production and expansion of specific T-cells.

In theory, autoimmune diseases can be prevented by blocking activation of T cells and formation of autoreactive T cells. Accordingly, there are a number of studies being conducted to identify methods or agents that can be used to block activation of T cells Unfortunately, since patients with autoimmune diseases have already developed autoreactive T cells, these methods have limited value for treatment of autoimmune diseases. Moreover, agents that prevent systemic T cell activation often cause serious side effects. For example, treatment with agents that block activation of T cells can also render the patient more susceptible to infections and cancer. Thus, it is desirable to have new methods for treating autoimmune diseases. A method which reduces the destruction of targeted tissues that is initiated by autoreactive T cells is especially desirable.

Previous studies have investigated the role of CD24/HSA (heat stable antigen) in the immune system. Chen et al., (Clin Exp Immunol, 1997, 108: 52-57) investigated the effect of HSA on activation of CD4⁻/CD8⁻ T cells. Enk et al., (Journal of Immunology, 1994, 152:3264) studied the effect of HSA expressed on epidermal Langerhans cells in cutaneous immune reactions and Saloga et al., (Exp Dermatol, 1995, 4: 291-296) performed a similar study using keratinocytes. Transgenic mice lacking functional HSA been investigated by Wenger et al., (Transgenic Research, 1995, 4: 173-183), and the research of Pawliuk et al., (Blood, 1994, 84: 2868-2877) has used mice expressing human CD24.

### Summary of the Invention

The present invention provides means for blocking autoreactive T cell-initiated destruction of tissues in a mammal as defined in the claims. In one embodiment, the invention comprises a pharmaceutical composition comprising a biologically effective amount of a fusion protein comprising human HSA/CD24 polypeptide for administering to a mammalian subject who is suspected of having multiple sclerosis, as defined in the claims. As used herein the term HSA/CD24 refers not only to the protein portion of the heat stable antigen (HSA) found on the surface of mouse cells but also to the mammalian homologs of mouse HSA. Thus, the term HSA/CD24, was used in the present application, encompasses the polypeptides portion of human CD24 and rat CD24, the known human and rat homologs of mouse HSA. The invention relates to human HSA/CD24 Preferably, the HSA/CD24 polypeptide is glycosylated. The fusion protein comprises the HSA/CD24 polypeptide or a truncated form of the HSA/CD24 linked by a peptide bond to a peptide or protein tag as defined in the claims.

### Brief Description of the Figures

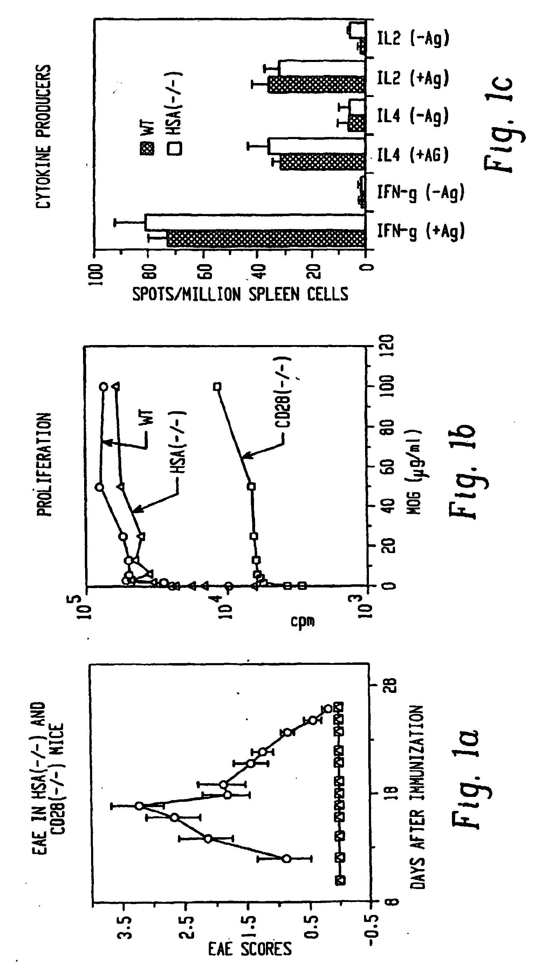

**Figure 1**. Targeted mutations of HSA and CD28 reveal two distinct checkpoints in the development of EAE. a. Targeted mutations of either HSA or CD28 prevent induction of EAE. WT, CD28(-/-) or HSA(-/-) mice were immunized with MOG peptide. Clinical signs were scored as described in the method section. b. Proliferative response of lymph node T cells to MOG peptides. Draining lymph node cells from day 10-immunized mice were stimulated with given concentrations of MOG peptide and irradiated syngeneic naive spleen cells as antigen-presenting cells. c. Enumeration of cytokine-producers by ELISpot. Draining lymph node cells used in b were used as responder cells. The numbers of cells secreting either IL2, IL4, and IFNγ among 1x10⁶ lymph node cells in response to MOG peptide (AA35-55) were presented. Data shown were means +/- SEM from three independent experiments.

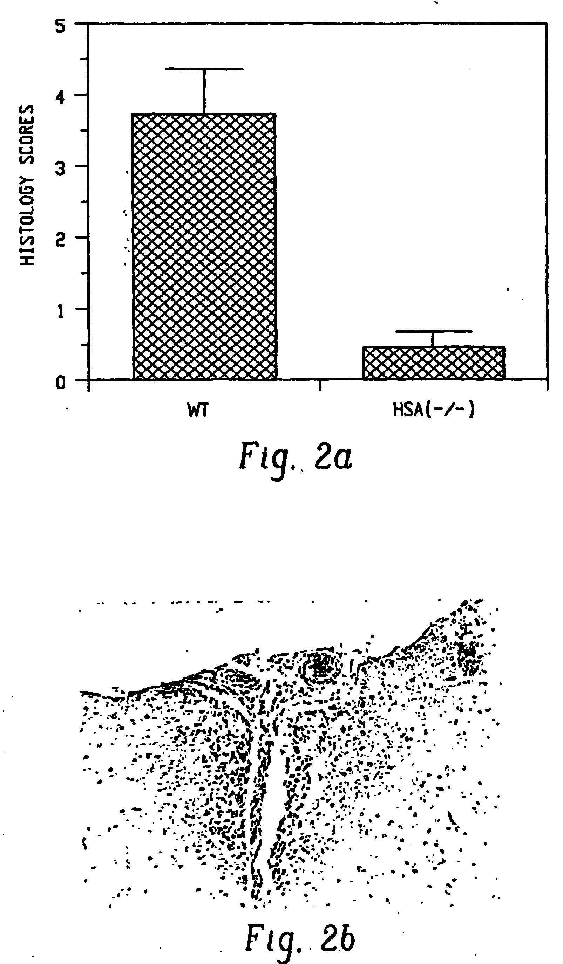

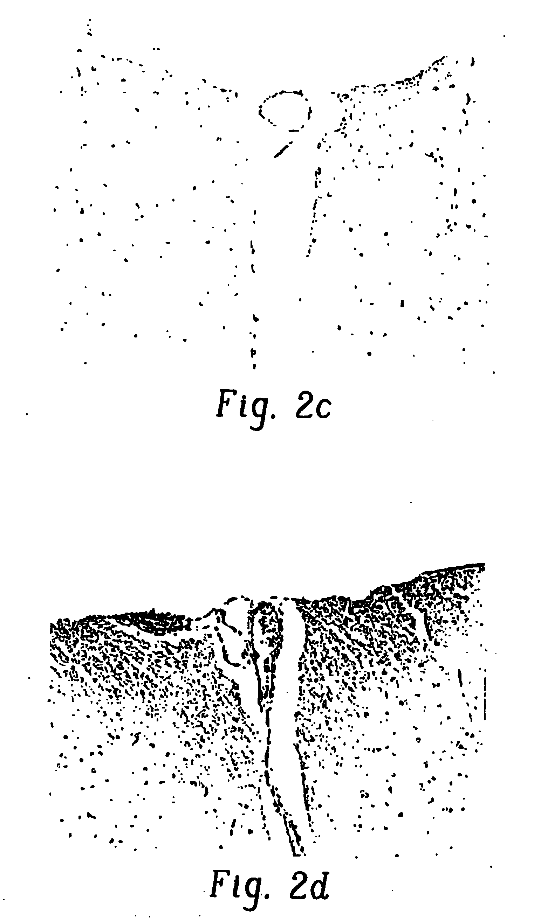

**Figure 2**. Histological analysis of spinal cord of MOG immunized WT or HSA(-/-) mice. a: The means and SEM of histological scores of WT and HSA(-/-) mice spinal cords. Ten independent cross sections, from cervical to sacral regions, were examined in each spinal cord. The data are summarized from 30 spinal cord sections from 3 mice in each group. b. Representative histology in immunized WT mice, all sections examined contain histology lesions. c and d. Histology sections (100x) of immunized HSA(-/-) mice. A lesion-free section is presented in c, while a lesion containing section is presented in d.

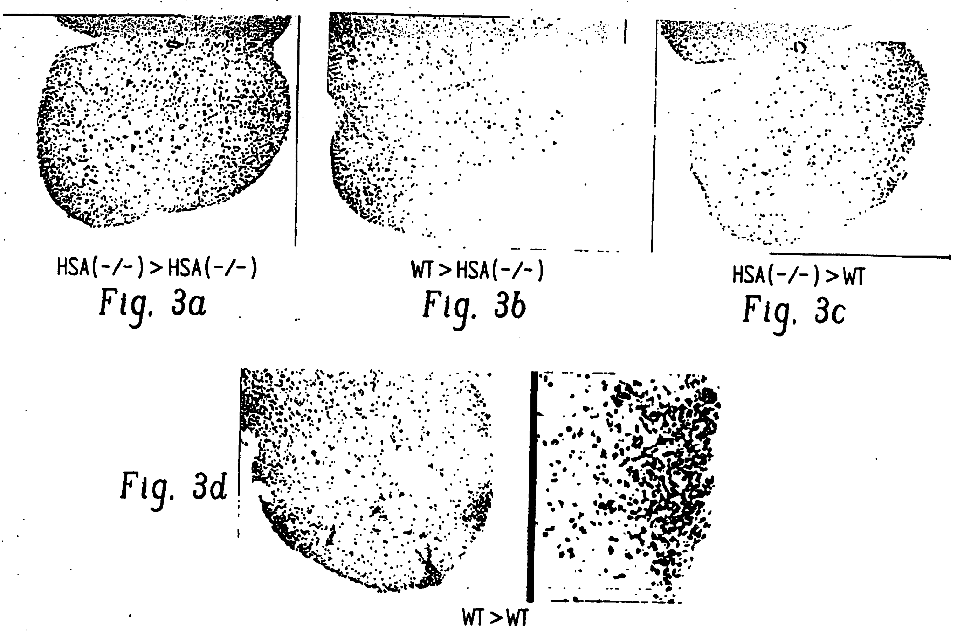

**Figure 3**. Requirement for HSA expression on both T cells and non-T host cells for the induction of EAE. Histology (63 x for a, b, c and the lcft panel of d; 200x for the right panel of d) of spinal cords of the HSA(-/-)(a, b) or WT(c, d) recipient mice on day 12 after adoptive transfer. Draining lymph node cells were isolated from either WT or HSA(/-) mice after immunization, and were stimulated with antigen and IL2 for 4 days in vitro. The activated T cells were injected into either WT or HSA(-/-) mice (100x10⁶ cells per mouse). EAE development was monitored daily for clinical signs. At 12 days after transfer, recipient mice were sacrificed and spinal cords were processed for histological examination. No disease was observed in WT>HSA(-/-), HSA(-/-)>WT, or HSA(-/)>HSA(-/-) recipients.

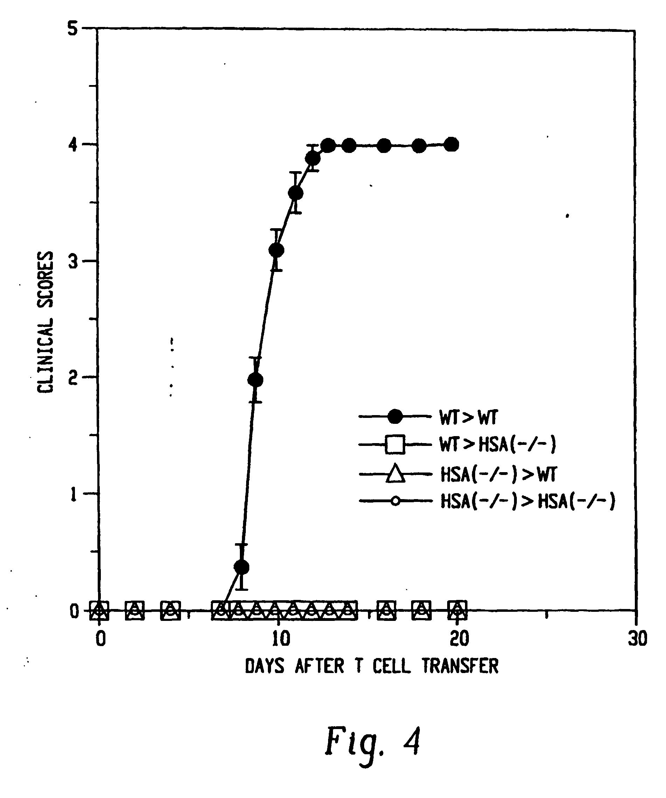

**Figure 4**. Clinical scores of the adoptive transfer experiment with 4 (WT>HSA(-/-) and HSA(-/-)>WT groups) or 5 (WT>WT and HSA(-/-)> HSA(-/-) groups) mice per group.

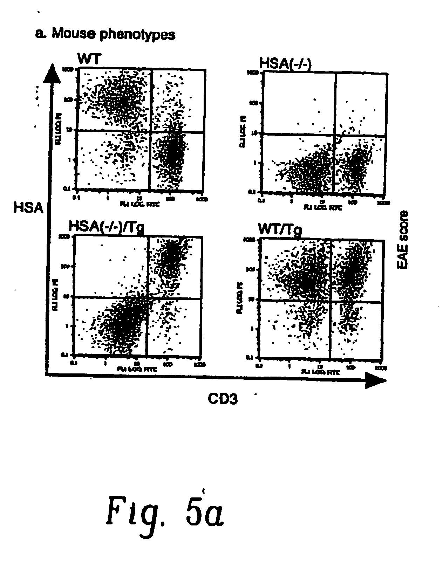

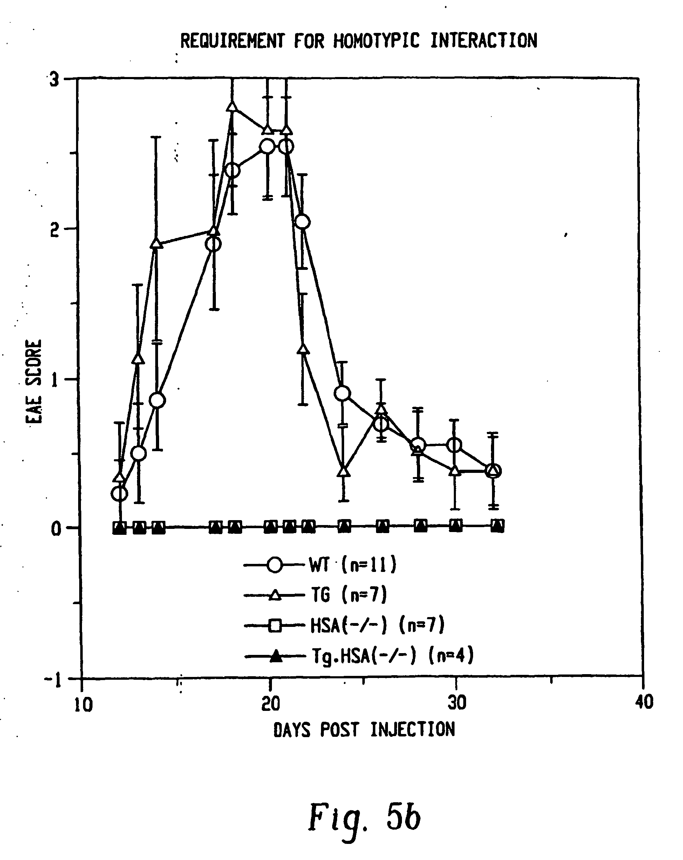

**Figure 5**. Transgenic expression of HSA exclusively on T cell lineage is insufficient for EAE development. a. Phenotypes of WT, HSA-TG, HSA(-/-), and HSATG/HSA(-/-) mice by flow cytometry using anti-HSA and anti-CD3 mAbs. b. EAE score in WT, HSATG, HSA(-/-), and HSATG/HSA(-/-) mice after immunization with the MOG peptides.

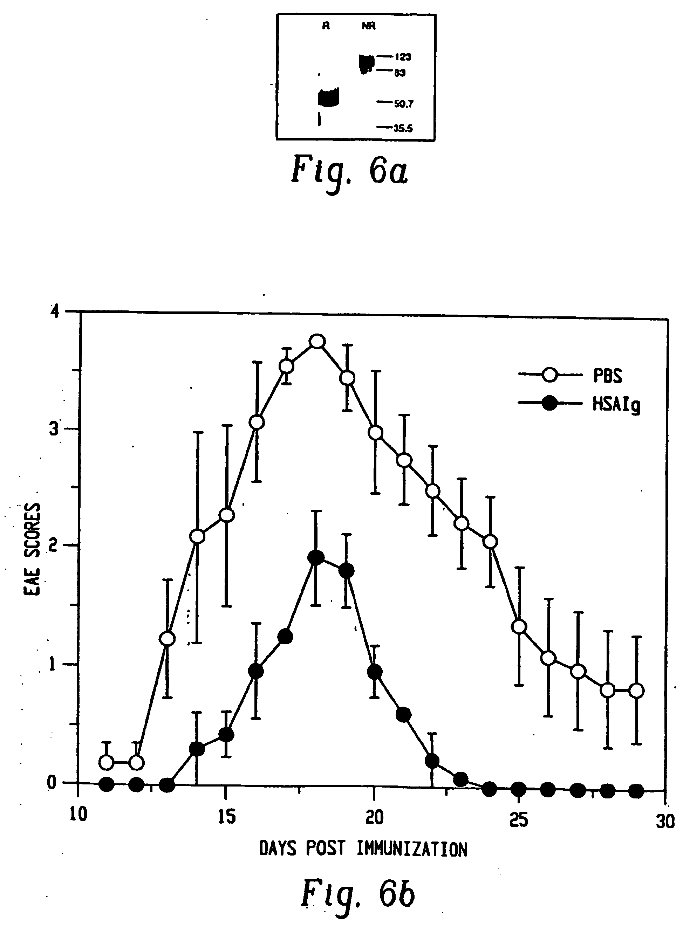

**Figure 6**. HSAIg ameliorates EAE. a. Analysis of HSAIg by SDS-PAGE. 10 µg of purified HSAIg was separated by 10% reducing (R) and non-reducing SDS-PAGE. The proteins were stained by Comassie blue. The EAE score for control (PBS) or HSAIg-treated mice. EAE was induced in WT mice as described in Materials and Methods.
   On days 8, 10, 12 , 14 and 22 after immunization, five mice per group were injected (i.p.) with 100 µg/mouse of either HSAlg or 100 ml of PUBS as control. The effect of HSAIg has been evaluated in three independent experiments with similar results.

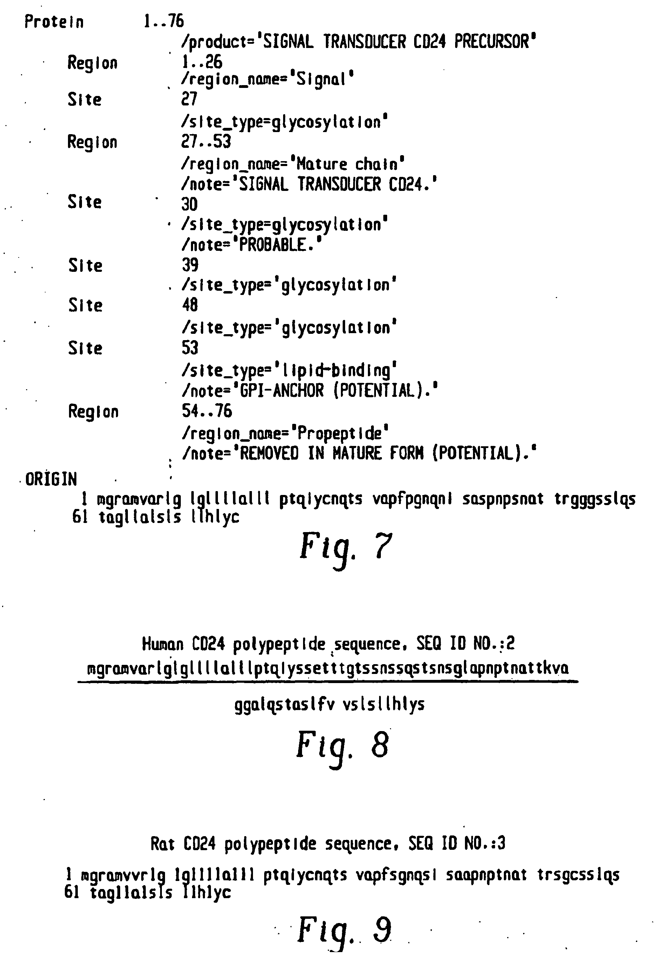

**Figure 7** shows the amino acid sequence, SEQ ID NO.1, of the mouse HSA polypeptide. The signal peptide extends from amino acid 1 through amino acid 26 of the sequence. The glycophosphatidyl (GPI) anchor region includes and extends from amino acid 54 through amino acid 76.
**Figure 8** shows the amino acid sequence, SEQ ID NO. 2, of the human CD24 polypeptide. The signal peptide extends from amino acid 1 through amino acid 26 of the sequence. The glycophosphatidyl (GPI) anchor region includes and extends from amino acid 60 through amino acid 80.
**Figure 9** shows the amino acid sequence, SEQ ID NO.3, of the rat CD24 polypeptide. The signal peptide extends from amino acid 1 through amino acid 26 of the sequence. The glycophosphatidyl (GPI) anchor region includes and extends from amino acid 57 through amino acid 76.

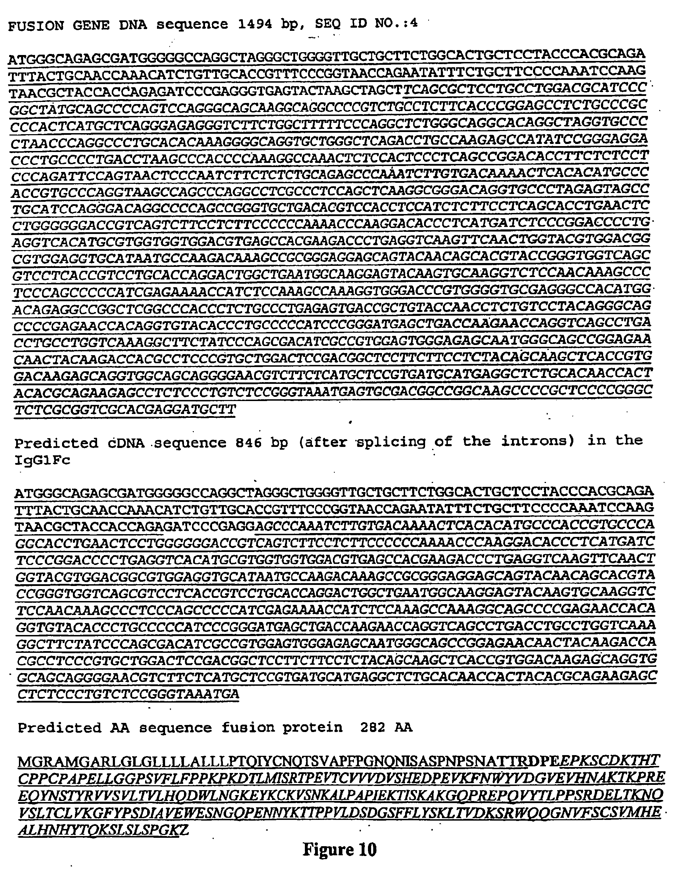

**Figure 10** shows the DNA sequence, SEQ ID NO.4, of a fusion gene which comprises a nucleotide sequence encoding HSA fused to the genomic sequence of human IgG1 Fc. The predicted sequence of the cDNA, SEQ ID NO. 5, which results from splicing of the introns IgG1 Fc sequence and the predicted amino acid sequence, SEQ ID NO. 14, are also shown in this figure. The normal font with under line is HSA sequence, bold phase is new sequence, italics is IgG1 Fc sequence.

### Detailed Description of the Invention

The present invention provides means for blocking destruction of tissue by autoreactive T cells in a mammalian subject as defined in the claims. One embodiment of the invention, as defined in the claims is a fusion protein comprising the human HSA/CD24 polypeptide or fragment thereof linked by a peptide bond to human IgG1 Fc, for administering to the mammal as defined in the claims. Preferably, the HSA/CD24 polypeptide is glycosylated.

The present invention also relates to agents for use in a method of treatment of a human subject suspected of having an autoimmune disease as defined in the claims. In one embodiment the method comprises administering a pharmaceutical composition comprising a biologically effective amount of the fusion protein to the human subject. Preferably, the pharmaceutical composition is for administering after autoreactive T cells have been detected in the human subject.

Preferably, the pharmaceutical composition is for administering by intravenous injection. The present invention is useful for treating subjects suspected of having multiple sclerosis (MS). By "treating" is meant ameliorating or tempering the severity of the condition. In cases of MS, the pharmaceutical composition is administered either when patients have clinical symptoms, or when they are in temporary remission. Preferably, the protocol involves intravenous injection.

### Pharmaceutical Composition

The pharmaceutical composition comprises a biologically effective amount of an HSA polypeptide or fragment thereof, and preferably a relatively inert topical carrier. Many such carriers are routinely used and can be identified by reference to pharmaceutical texts.

### HSA Antigen

The mouse HSA antigen and the mammalian homologs thereof are polypeptides comprising approximately 76 amino acids. The HSA polypeptide and the mammalian homologs thereof are cell surface molecules which are linked to the cell membrane via a glycophosphatidylinositol (GPI) tail. The HSA antigen is constitutively expressed on most hematopoietic and developing neuronal cells. In some lymphocytes, such as for example T cells, expression of the HSA polypeptide is induced. As shown in Figures 7-9, the immature forms of mouse H antigen, human CD24 and rat CD24 comprise a signal sequence, a core region that is maintained in the mature protein, and a GPI anchor region. The nucleotide sequence of a polynucleotide which encodes the human CD4 polypeptide is available at the GenBank Accession No. AK000168. The nucleotide sequence of a cDNA which encodes the rat CD24 polypeptide is available at GenBank Accession No. AWK12164. The nucleotide sequence of a cDNA which encodes mouse HSA antigen is available at GenBank Accession M58661.

The present invention relates to an HSA/CD24 polypeptide or fragment thereof for use in a method of treating autoimmune diseases. Preferably, the polypeptide or fragment is glycosylated. In one embodiment the HSA/CD24 fragment is a truncated form of the HSA/CD24 polypeptide which lacks a few amino acids, i.e., from 1 to 2 amino acids, at the amino terminus or carboxy terminus thereof. In another embodiment the HSA/CD24 fragment is a polypeptide which comprises only the core region of the HSA/CD24 polypeptide, i.e. the HSA/CD24 fragment lacks the entire signal peptide and GPI anchor region. As used herein the term HSA/CD24 polypeptide comprises all mammalian homologs of mouse HSA , including human CD24 and rat CD24; the invention is limited, as defined in the claims, to fusion proteins comprising human CD24 or fragments thereof.

Preferably, the HSA/CD24 polypeptide or HSA/CD24 fragment that is used in the pharmaceutical composition has an amino acid sequence which is at least 80%, more preferably at least 93%, most preferably at least 96% identical to the amino acid sequence of the naturally occuring HSA/CD24 polypeptide or HSA/CD24 that is present in the mammal that the pharmaceutical composition is for administering to. For murine HSA, alteration in Postions 1(Asn), 4(Ser), 13(Asn), 15(Ser), 17(Ser), 21(Ser), 22(Asn), 24(Thr) and 25(Thr) of the mature peptide, i.e., the peptide which lacks the signal peptide, may alter glycosylation and interfere with its ability to block destruction of tissue by autoreactive T cells. Thus, it is preferred that alternations not be made at these sites. In the human homologue of HSA, i.e., human CD24, 20 out of 31 amino acids are potential glycosylation sites.

An HSA/CD24 polypeptide which is less than 100% identical to the naturally occurring HSA/CD24 polypeptide has an altered sequence in which one or more of the amino acids in the HSA homologue is deleted or substituted, or one or more amino acids are inserted into the sequence of the naturally occurring HSA/CD24 polypeptide. HSA/CD24 sequences which are at least 95% identical to the naturally occurring HSA/CD24 sequence have no more than 5 alterations, i.e., any combination of deletions, insertions or substitutions, per 100 amino acids of the reference sequence. Percent identity is determined by comparing the amino acid sequence of the altered HSA/CD24 sequence with the naturally occurring sequence using MEGALIGN project in the DNA STAR program. Sequences are aligned for identity calculations using the method of the software basic local alignment search tool in the BLAST network service (the National Center for Biotechnology Information, Bethesda, MD) which employs the method of Altschul, S.F., Gish, W., Miller, W., Myers, E. W. & Lipman, D. J. (1990) J. Mol. Biol. 215, 403-410. Identities are calculated by the Align program (DNAstar, Inc.) In all cases, internal gaps and amino acid insertions in the candidate sequence as aligned are not ignored when making the identity calculation.

While it is possible to have nonconservative amino acid substitutions, it is preferred that the substitutions be conservative amino acid substitutions, in which the substituted amino acid has similar structural or chemical properties with the corresponding amino acid in the reference sequence. By way of example, conservative amino acid substitutions involve substitution of one aliphatic or hydrophobic amino acids, e.g. alanine, valine, leucine and isoleucine, with another; substitution of one hydroxyl-containing amino acid, e.g. serine and threonine, with another; substitution of one acidic residue, e.g. glutamic acid or aspartic acid, with another; replacement of one amide-containing residue, e.g. asparagine and glutamine, with another; replacement of one aromatic, residue, e.g. phenylalanine and tyrosine, with another; replacement of one basic residue, e.g. lysine, arginine and histidine, with another; and replacement of one small amino acid, e.g., alanine, serine, threonine, methionine, and glycine, with another.

The alterations are designed not to abolish or substantially reduce the ID₅₀ of the HSA/CD24 polypeptide or fusion protein comprising such polypeptide in suppressing the clinical symptom of experimental autoimmune models, such as EAE or Type II diabetes in NOD mouse or rat. Alternatively, one can determine the ID₅₀ in an adhesion assay. The amount of the variant needed to reduce binding of activated T cells to vascular endothelial cells by at least 50%, preferably, is no greater than twice the amount of the naturally occurring HSA/CD24 polypeptide.

The present invention comprises fusion proteins comprising an HSA/CD24 polypeptide or the core region thereof linked by a peptide bond to the hinge-CH2-CH3 regions of human immunoglobin G1 ("IgG1"), which is added to the amino terminus of or the carboxy terminus of the amino acid sequence of the HSA/CD24 polypeptide or core region thereof. Peferably, the HSA/CD24 polypeptide or core region thereof is glycosylated. Typically, such additions are made to simplify purification of an expressed recombinant form of the corresponding HSA/CD24 polypeptide or core region thereof. Such tags are known in the art. The fusion protein can be easily purified by affinity chromatography using either anti-IgG or protein A or protein G. Since IgG is not immunogenic in humans; the fusion protein can be administrated repeatedly if necessary.

### Methods of Preparing the HSA/CD24 Fusion Protein

The HSA/CD24 fusion proteins may be produced by using cell-free translation systems and RNA molecules derived from DNA constructs that encode the polypeptide or fusion protein. Preferably, the HSA/CD24 fusion protein is made by transfecting host cells with expression vectors that comprise a DNA sequence that encodes the respective HSA/CD24 fusion protein and then inducing expression of the polypeptide in the host cells. For recombinant production, recombinant constructs comprising one or more of the sequences which encode the HSA/CD24 fusion protein are introduced into host cells by conventional methods such as calcium phosphate transfection, DEAE-dextran mediated transfection, transvection, microinjection, cationic lipid-mediated transfection, electroporation, transduction, scrape lading, ballistic introduction or infection.

The HSA/CD24 fusion protein may be expressed in suitable host cells, such as for example, mammalian cells, yeast, insect cells or other cells under the control of appropriate promoters using conventional techniques. Suitable hosts include, but are not limited to, CHO, COS cells and 293 HEK cells. Following transformation of the suitable host strain and growth of the host strain to an appropriate cell density, the cells are harvested by centrifugation, disrupted by physical or chemical means, and the resulting crude extract retained for further purification of the epitope or chimeric peptide. For obtaining properly glycosylated forms of the protein, it is preferred that CHO cells be used.

Conventional procedures for isolating recombinant proteins from transformed host cells, such as isolation by initial extraction from cell pellets or from cell culture medium, followed by salting-out, and one or more chromatography steps, including aqueous ion exchange chromatography, size exclusion chromatography steps, and high performance liquid chromatography (HPLC), and affinity chromatography may be used to isolate the recombinant polypeptide.

### Carrier

The acceptable carrier is a physiologically acceptable diluent or adjuvant. The term physiologically acceptable means a non-toxic material that does not interfere with the effectiveness of HSA. The characteristics of the carrier will depend on the route of administration and particular compound or combination of compounds in the composition. Preparation of such formulations is within the level of skill in the art. The composition may further contain other agents which either enhance the activity of the HSA or complement its activity. The composition may further comprise fillers, salts, buffers, stabilizers, solubilizers, and other materials well known in the art.

### Dosage

A biologically effective amount is an amount sufficient to partially or completely block destruction of the targeted tissue initiated by the autoreactive T cell or to ameliorate the pathological effects of the autoimmune disease. The effective amount can be achieved by one administration of the composition. Alternatively, the effective amount is achieved by multiple administration of the composition to the mammal.

The invention further provides a pharmaceutical composition which comprises a biologically non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321: 522-525 (1986); Reichmann et al., Nature 332: 323-329 (1988); and Presta, Curr. Op. Struct. Biol. 2: 593-596 (1992).

Alternatively, transgenic mice with human IgV and IgC genes may be used to produce human mAb specific for human CD24. These mice are available from Obgenix, and the art has been described fully (Nature Genetics, 1997, 15: 146).

### Transgenic Models to Test the Effect of CD24 Blockers In Vivo

Since the major cell type in the CNS that expresses HSA is the brain vascular endothelial cells, transgenic vectors that give specific expression of human CD24 in both T cells and vascular endothelial cells are used to prepare the transgenic mice. In one preferred embodiment T cell-specific expression is achieved using a transgenic vector comprised of human CD24 open reading frame and the proximal lck promoter and vascular endothelial cell specific expression is achieved using a transgenic vector comprised of human CD24 open-reading frame and the Tie II promoter, as described in Proc. Natl. Acad Sci USA. 94:3058-63(1997). To avoid interference by the endogenous HSA, the transgenic vector is injected into the fertilized embryos from mice with a targeted mutation of mouse CD24 as described in J. Exp. Med. 85:251-262(19985). Alternatively, the transgenic mice expressing the CD24 gene can be bred to the CD24 (-/-) mice to avoid expression of endogenous CD24. The tissue specificity of the transgene expression is verified with anti-CD24 mAb, which is available from Pharmingen (San Diego, CA), by flow cytometry and immunhistochemistry according to established procedure.

The transgenic mice produced as described above are used to screen drugs targeted at the human CD24 molecules. One example is to screen for drugs which inhibit or ameliorate autoimmune conditions such as multiple sclerosis. The most suitable murine model for multiple sclerosis is EAE, which is induced by immunizing mice with MOG according to an established procedure.

### Example 1 Treatment of Animals with Experimental Autoimmune Encephalomyelitis with HSAIg

### Methods

Wild type C57BL/6 mice (WT) were purchased from the National Cancer Institute (Bethesda, MD). Mice homozygous for the disrupted HSA (produced with ES cells from C57BL/6 mice) (18) (24) or CD28 (25) (backcrossed to C57BL/6 for more than 8 generation) locus have been described before and are maintained at the animal facilities of the Ohio State University Medical Center. HSA transgenic mice (HSATG) have been described previously (See Zhou, Q., Wu, Y., Nielsen, P.J., and Liu, Y. 1997. Homotypic interaction of the heat-stable antigen is not responsible for its co-stimulatory activity for T cell clonal expansion. Eur J Immunol, 27:2524-2528) and have been backcrossed to C57BL/6j background for more than 5 generations. Mice with HSA exclusively expressed on the T cell lineage (HSATG/HSA(-/-)) were generated by crossing HSATG with the HSA(-/-) mice.

*Induction and clinical evaluation of EAE* The immunogen, MOG peptide 35-55 of rat origin (MEVGWYRSPFSRVVHLYRNGK), was synthesized by Research Genetics, Inc. (Huntsville, AL, USA). The purity of the peptide, was >90%. Mice of 8-12 wks of age were immunized subcutaneously with 200 µg MOG peptide in complete Freund's Adjuvant (400 µg *of Mycobacterium tuberculosis* per ml) in a total volume of 100 µl. They received 200 µg of Pertusis toxin (List Biological, Campbell, CA) in 200 µl PBS in the tail vein immediately after the immunization, and again 48 hours later. The mice were observed every other day and scored on a scale of 0-5 with gradations of 0.5 for intermediate scores: 0, no clinical signs; 1, loss of tail tone; 2, wobbly gait; 3, hind limb paralysis; 4, hind and fore limb paralysis; 5, death.

T cell proliferation assay Draining lymph node cells were isolated 10 days after immunization. 5x10⁵ cells/well were stimulated with given concentrations of MOG peptide in the presence 6x10⁵ cells/well of irradiated (2,000 rad) syngeneic splenocytes for 60 hours. The cultures were pulsed with ³H-thymidine (1 µCi/well; ICN Pharmaceuticals Inc., Costa Mesa, CA USA) for another 12 hours, and incorporation of 3H-thymidine was measured in a liquid scintillation P-plate counter.

*ELISpot* assay to evaluate *frequencies of T cells* that produce *IFN-γ, IL-2 and IL-4 upon restimulation* with MOG peptide in vitro The antibody pairs and the procedures have been described (20), except that the MOG peptide was used for stimulation at 10 µg/ml. The numbers presented are those of cytokine producers per million of draining lymph node cells. Histology

Mice were sacrificed by CO₂ inhalation. Spinal cords were removed by insufflation and fixed in 10% formalin/PBS. Paraffin sections were prepared and stained with hematoxylin and eosin. Neurological lesions were graded on each of the 10 cross sections per spinal cord, according the following criteria: 0, no infiltrate; 1, 3 or less focal meningeal infiltrates; 2, more than 3 focal meningeal infiltrates; 3, up to 5 perivascular infiltrate foci in the parenchyma with involvement of less than 5% of the white matter; 4, 5-10 perivascular foci in the parenchyma or invasions involving 5-25% the white matter; 5, more than 10 perivascular foci or diffuse infiltration involving more than 25% of the white matter.

### Passive transfer of EAE

Groups of 8-10 WT and HSA(-/-) mice were immunized with 200 µg of MOG peptide subcutaneously. At 10 days after immunization, draining lymph nodes were harvested and stimulated at 4 x 10⁶/ml in Click's EHAA medium supplemented, with 15% fetal calf sera, 5% IL-2 supernatant, and 50 µg/ml of MOG peptide for 4 days. 1 x 10⁸ cells were injected i.p. into each recipient mouse that had been γ-irradiated (550 rad) 1 h earlier.

### Preparation of fusion protein and treatment of EAE z

The HSA fragment encoding the signal peptide and the mature protein sequence were amplified by PCR, using GGA AAG CTT ATG GGC AGA GC, SEQ ID NO.:6, as forward primer, CGA GAT CTC TGG TGG TAG CG , SEQ ID NO.:7, as reverse primer, and HSA cDNA as template. The PCR products were digested with *Hind III* and Bgi II enzymes and were ligated to *Hind III* and *Xba* I-digested pCDM8 vector (Invitrogen, San Diego) and a *Xba* I and *Bam* HI-treated DNA fragment encoding human IgG1 Fc, which were amplified by PCR using CAG GGA TCC CGA GGG TGA GTA CTA AGC TAG CTT CAG CGC TCC TGC CTG, SEQ ID NO.:7, as forward primer and CTT CGA CCA GTC TAG AAG CAT CCT CGT GCG ACC GCG AGA GC, SEQ ID NO.:8, as reverse primer, and DNA from human peripheral blood as template. The construct was verified by DNA sequencing and was used to transfect the Chinese Hamster Ovary cell line. The cells that secreted HSAIg fusion protein were amplified in DMEM containing 5% fetal calf serum until confluence. The cell monolayers were washed with serum-free medium and cultured in optimal M medium for 72 hours. The supernatants were collected and the HSAIg was purified using a protein G column according to the manufacturer's protocol. The purity of the protein was verified by SDS PAGE.

### Results

To test if HSA is essential for the development of EAE, we immunized C57BL/6 wild-type (WT), and HSA- or CD28-deficient mice with myelin oligodendrocyte glycoprotein (MOG) peptide AA35-55 in conjunction with complete Freund's adjuvant and pertusis toxin. As shown in Fig. 1a, wild-type mice developed acute EAE within two weeks of peptide immunization, while those with targeted mutation of either HSA or CD28 were completely resistant to EAE induction. Interestingly, while targeted mutation of CD28 ablated induction of MOG-specific T cells, as revealed by proliferative response of draining lymph node cells, that of HSA had little effect on peptide-specific T cell proliferation (Fig. lb). Moreover, the frequencies of antigen-specific, IL2-, IL4-, and IFNγ-producing cells were not altered in HSA(-/-) mice (Fig. 1c). The anti-MOG peptide IgG responses were also detected in HSA-deficient mice (data not shown). The differential effects of HSA and CD28 mutations on T cell priming reveal that these genes mediate two distinct checkpoints in the development of EAE: CD28 controls induction of auto-reactive T cells, while HSA determines their pathogenicity.

Histological analysis of MOG-peptide immunized WT arid HSA- confirms the clinical scores. The histological scores were summarized in Fig. 2a, while representative histology sections were presented Fig. 2b-d. As shown in Fig. 2b, active immunization with MOG peptide induces multiple neurological lesions in the wild-type mice, characterized by multiple lesions with extensive invasion of parenchyma. In contrast, the spinal cords of HSA-KO mice are either devoid of any lesion (Fig. 2c), or with one or two low grade lesions involving meninges (Fig. 2d).

We adoptively transferred activated draining lymph node cells to WT and HSA-deficient recipients. As shown in Fig. 3 and 4, WT T cells induced severe EAE in WT recipients within 8 days of adoptive transfer. Interestingly, none of the HSA-deficient recipients developed EAE. Thus HSA expression on T cells alone appears insufficient for EAE development. Moreover, T cells from HSA-deficient mice failed to induce disease regardless of HSA gene status in the recipient, which indicates that HSA expression on T cells is necessary for EAE development. These results strongly suggest that HSA must be expressed on both host cells and auto-reactive T cells in order to induce EAE.

To substantiate these observations, we produced mice that expressed HSA exclusively on T cells. We have previously reported the transgenic mice in which expression of HSA was under the control of the *lck* proximal promoter (HSATG) (22). For this study, we crossed the HSA transgene to HSA-deficient mice to produce mice that expressed HSA exclusively on T cells (Fig. 5a). To test if HSA expression on the T cell lineage is sufficient for EAE development, we immunized WT, HSA-TG, HSA(-/-) and HSATG HSA(-/-) mice with MOG. As shown in Fig. 5b, wild-type and HSATG mice developed EAE with essentially identical kinetics, which indicates that transgenic expression of HSA on T cells does not prevent the production and effector function of self-reactive T cells. Nevertheless, much like HSA (-/-) mice, the mice with exclusive HSA-expression on the T cell lineage failed to develop EAE. These results demonstrated clearly that HSA expression on T cell lineage alone is insufficient for EAE development.

The fact that HSA may be a critical checkpoint after activation of self-reactive T cells suggests a novel approach in treating autoimmune neurological diseases. Since an anti-HSA mAb was toxic in the EAE model to address this issue (Data not shown), we produced a fusion protein between the extracellular domain of HSA and the Fc portion of human IgG1, to block the HSA-mediated interactions. As shown in Fig. 6a, the fusion protein has an apparent molecular weight of about 100 kD under non-reducing SDS-PAGE. After reduction, it migrated as a 50 kD band. We treated mice starting at 8-10 days after immunization with MOG peptide, when MOG-specific T cells response had already expanded in the local lymph nodes. As shown in Fig. 6b, HSAIg drastically ameliorated EAE. All HSAIg-treated mice recovered substantially earlier than did the control mice. Since MOG-reactive T cells had been activated prior to HSAIg administration, the clinical signs in the treated group may reflect the fact that some autoreactive T cells had already migrated into the central nervous system.

HSAIg, a fusion protein consisting of the extracellular domain of mouse HSA and the Fc portion of immunoglobulin, drastically ameliorates the clinical sign of EAE even when administrated after self-reactive T cells had been expanded. Thus, identification of HSA as a novel checkpoint, even after activation and expansion of self-reactive T cells, provides a novel approach for immunotherapy of autoimmune neurological diseases, such as multiple sclerosis.

### Example 2 Production Human CD24Ig fusion protein

Fragments of the human CD24 polypeptides lacking the GPI anchor region are fused with human Ig constant region to form CD24-Ig fusion protein. In one embodiment the CD24 polypeptide fragment comprises the signal peptide. In another embodiment the CD24 polypeptide fragment lacks the signal peptide. The fragment of the human CD24 coding sequence is subcloned into vector pIg (from Novagen) Hind III and BamHI sites. Suitable primers useful in subcloning include, but are not limited to, CD24 forward primer (CD24F.H3): G GCC AAG CTT ATG GGC AGA GCA ATG GTG, SEQ ID NO.:9, with Hind III site 5' to ATG start codon. CD24-Ig reverse primer (CD24Rig.Bm): GG CCG GAT CCA CTT ACC TGT CGC CTT GGT GGT GGC ATT, SEQ ID NO.10, with Bam HI site and the SD sequence (A CTT ACC TGT, SEQ ID NO.:11) next to 3' end of TTKA (direct sequence: ACC ACC AAG GCG, SEQ ID NO.:12) in Human CD24. The construct is transfected into CHO cells, and the CD24Ig is secreted into the tissue culture medium. CD24Ig is purified by affinity chromatography using a Protein G column. The clone compresses CD24 signal peptide, CD24 core peptide and the IgG/Fc portion, but lacks the GPI anchor signaling region.

### Example 3 Production of anti-human CD24 mAb that blocks autoreactive T cells-initiated tissue destruction

Human CD24 coding sequence is subcloned into vector pCDM8 (from Invitrogen) Hind III and Xho I sites. CD24 forward primer (CD24F.H3): G GCC AAG CTT ATG GGC AGA GCA ATG GTG with Hind III site 5' to ATG start codon. CD24 reverse primer (CD24R. Xho): A TCC CTC GAG TTA AGA GTA GAG ATG CAG with Xho I site 3' to TAA stop codon. The CD24 cDNA is transfected into murine 3T3 cells. The 3T3 cell lines that stably express human CD24 molecules are used to immunize syngeneic mice. After 2-3 immunizations spleen cells are fused with myeloma AgX865, after selection with HAT medium the supernatants are screened for anti-human CD24 mAbs. The antibodies are tested for their ability to block both adhesion of human T cells to human endothelial cells in vitro, and their ability to block human CD24-mediated T cell trafficking to target tissues, such as the pancreas and the central nervous system using the transgenic model detailed below.

### Example 4 Testing Putative Inhibitors of Multiple Sclerosis with Transgenic Mice

The immunogen, MOG peptide 35-55 of rat origin (MEVGWYRSPFSRVVHLYRNGK, SEQ ID NO.:13), is available from Research Genetics, Inc. (Huntsville, AL, USA). Mice of 8-12 wks of age are immunized subcutaneously with 200 µg MOG peptide in complete Freund's Adjuvant (400 µg of *Mycobacterium tuberculosis* per ml) in a total volume of 100 µl. They receive 200 µg of Pertusis toxin (List Biological, Campbell, CA) in 200 Id PBS in the tail vein immediately after the immunization, and again 48 hours later. The mice are observed every other day and scored on a scale of 0-5 with gradations of 0.5 for intermediate scores: 0, no clinical signs; 1, loss of tail tone; 2, wobbly gait; 3, hind limb paralysis; 4, hind and fore limb paralysis; 5, death. The putative inhibitory molecules are injected at 1 week after immunization. Those that substantially reduce the clinical score of EAE are selected for further testing.

## Claims

1. An HSA/CD24 fusion protein for use in a method of treating a human subject suspected of having multiple sclerosis, wherein the HSA/CD24 fusion protein comprises human HSA/CD24 polypeptide, or a fragment thereof comprising the core region, linked by a peptide bond to human IgG1 Fc.

2. The HSA/CD24 fusion protein for use according to claim 1, wherein the fragment lacks the GPI anchor region and signal peptide.

3. The HSA/CD24 fusion protein for use according to claim 1, wherein the fragment lacks the GPI anchor and comprises the signal peptide.

4. The HSA/CD24 fusion protein for use according to any preceding claim, wherein the HSA/CD24 polypeptide or fragment thereof is glycosylated.

5. The HSA/CD24 fusion protein for use according to any preceding claim, which is produced by expression in a mammalian host cell.

6. The HSA/CD24 fusion protein for use according to claim 5, wherein the host cell is a CHO cell.

7. The HSA/CD24 fusion protein for use according to any preceding claim, which is for use in the treatment of a human subject having clinical symptoms of multiple sclerosis.

8. The HSA/CD24 fusion protein for use according to any one of claims 1 to 6, which is for use in the treatment of a human subject in temporary remission from symptoms of multiple sclerosis.

9. Use of an HSA/CD24 fusion protein in the manufacture of a medicament for treating a human subject suspected of having multiple sclerosis, wherein the HSA/CD24 fusion protein comprises human HSA/CD24 polypeptide, or a fragment thereof comprising the core region, linked by a peptide bond to human IgG1 Fc.

10. The use of claim 9, wherein the fragment lacks the GPI anchor region and signal peptide.

11. The use of claim 9, wherein the fragment lacks the GPI anchor and comprises the signal peptide.

12. The use of any one of claims 9 to 11, wherein the HSA/CD24 polypeptide or fragment thereof is glycosylated.

13. The use of any one of claims 9 to 12, wherein the HSA/CD24 fusion protein is produced by expression in a mammalian host cell.

14. The use of claim 13, wherein the host cell is a CHO cell.

15. The use of any one of claims 9 to 14, wherein the medicament is for use in the treatment of a human subject having clinical symptoms of multiple sclerosis.

16. The use of any one of claims 9 to 14, wherein the medicament is for use in the treatment of a human subject in temporary remission from symptoms of multiple sclerosis.

## Patentansprüche

1. HSA/CD24-Fusionsprotein zur Verwendung in einem Verfahren zur Behandlung eines menschlichen Individuums mit Verdacht auf Multiple Sklerose, worin das HSA/CD24-Fusionsprotein menschliches HSA/CD24-Polypeptid oder ein Fragment davon, das die Kernregion umfasst, gebunden durch eine Peptidbindung an menschliches IgG1-Fc umfasst.

2. HSA/CD24-Fusionsprotein zur Verwendung nach Anspruch 1, worin dem Fragment die GPI-Ankerregion und das Signalpeptid fehlt.

3. HSA/CD24-Fusionsprotein zur Verwendung nach Anspruch 1, worin dem Fragment der GPI-Anker fehlt und es das Signalpeptid umfasst.

4. HSA/CD24-Fusionsprotein zur Verwendung nach einem der vorangegangenen Ansprüche, worin das HSA/CD24-Polypeptid oder das Fragment davon glykosyliert ist.

5. HSA/CD24-Fusionsprotein zur Verwendung nach einem der vorangegangenen Ansprüche, das durch Expression in einer Säugetierwirtszelle produziert ist.

6. HSA/CD24-Fusionsprotein zur Verwendung nach Anspruch 5, worin die Wirtszelle eine CHO-Zelle ist.

7. HSA/CD24-Fusionsprotein zur Verwendung nach einem der vorangegangenen Ansprüche, das der Verwendung bei der Behandlung eines menschlichen Individuums, das klinische Symptome von Multipler Sklerose aufweist, dient.

8. HSA/CD24-Fusionsprotein zur Verwendung nach einem der Ansprüche 1 bis 6, das der Verwendung bei der Behandlung eines menschlichen Individuums in vorübergehender Remission von Symptomen von Multipler Sklerose dient.

9. Verwendung eines HSA/CD24-Fusionsproteins zur Herstellung eines Medikaments zur Behandlung eines menschlichen Individuums mit Verdacht auf Multiple Sklerose, worin das HSA/CD24-Fusionsprotein menschliches HSA/CD24-Polypeptid oder ein Fragment davon, das die Kernregion umfasst, gebunden durch eine Peptidbindung an menschliches IgG1-Fc umfasst.

10. Verwendung nach Anspruch 9, worin dem Fragment die GPI-Ankerregion und das Signalpeptid fehlt.

11. Verwendung nach Anspruch 9, worin dem Fragment der GPI-Anker fehlt und es das Signalpeptid umfasst.

12. Verwendung nach einem der Ansprüche 9 bis 11, worin das HSA/CD24-Polypeptid oder das Fragment davon glykosyliert ist.

13. Verwendung nach einem der Ansprüche 9 bis 12, worin das HSA/CD24-Fusionsprotein durch Expression in einer Säugetierwirtszelle produziert ist.

14. Verwendung nach Anspruch 13, worin die Wirtszelle eine CHO-Zelle ist.

15. Verwendung nach einem der Ansprüche 9 bis 14, worin das Medikament der Verwendung bei der Behandlung eines menschlichen Individuums, das klinische Symptome von Multipler Sklerose aufweist, dient.

16. Verwendung nach einem der Ansprüche 9 bis 14, worin das Medikament der Verwendung bei der Behandlung eines menschlichen Individuums in vorübergehender Remission von Symptomen von Multipler Sklerose dient.

## Revendications

1. Protéine de fusion HSA/CD24 pour utilisation dans une méthode de traitement d'un sujet humain dont on suspecte qu'il a la sclérose multiple, ou la protéine de fusion HSA/CD24 comprend un polypeptide HSA/CD24 humain ou un fragment de celui-ci comprenant la région de coeur liée par une liaison peptide à la IgG1 Fc humaine.

2. Protéine de fusion HSA/CD24 pour utilisation selon la revendication 1, dans laquelle le fragment est exempt de la région d'ancrage de GPI et du peptide signal.

3. Protéine de fusion HSA/CD24 pour utilisation selon la revendication 1, où le fragment est exempt de l'ancrage de GPI et comprend le peptide signal.

4. Protéine de fusion HSA/CD24 pour utilisation selon l'une quelconque des revendications précédentes, où le polypeptide HSA/CD24 ou son fragment est glycosylé.

5. Protéine de fusion HSA/CD24 pour utilisation selon l'une quelconque des revendications précédentes, qui est produite par l'expression dans une cellule hôte mammalienne.

6. Protéine de fusion HSA/CD24 pour utilisation selon la revendication 5, où la cellule hôte est une cellule CHO.

7. Protéine de fusion HSA/CD24 pour utilisation selon l'une quelconque des revendications précédentes, qui est pour l'utilisation dans le traitement d'un sujet humain présentant des symptômes cliniques de sclérose multiple.

8. Protéine de fusion HSA/CD24 pour utilisation selon l'une quelconque des revendications 1 à 6, qui est pour l'utilisation dans le traitement d'un sujet humain en rémission temporaire des symptômes de la sclérose multiple.

9. Utilisation d'une protéine de fusion HSA/CD24 dans la fabrication d'un médicament pour le traitement d'un sujet humain dont on suspecte qu'il a la sclérose multiple, où la protéine de fusion HSA/CD24 comprend un polypeptide HSA/CD24 humain ou un fragment de celui-ci comprenant la région de coeur, lié par une liaison peptide à la IgG1 Fc humaine.

10. Utilisation selon la revendication 9, dans laquelle le fragment est exempt de la région d'ancrage de GPI et du peptide signal.

11. Utilisation selon la revendication 9, dans laquelle le fragment est exempt de l'ancrage de GPI et comprend le peptide signal.

12. Utilisation selon l'une quelconque des revendications 9 à 11, où le polypeptide HSA/CD24 ou son fragment est glycosylé.

13. Utilisation selon l'une quelconque des revendications 9 à 12, où la protéine de fusion HSA/CD24 est produite par expression dans une cellule hôte mammalienne.

14. Utilisation selon la revendication 13, où la cellule hôte est une cellule CHO.

15. Utilisation selon l'une quelconque des revendications 9 à 14, où le médicament est pour l'utilisation dans le traitement d'un sujet humain présentant des symptômes cliniques de sclérose multiple.

16. Utilisation selon l'une quelconque des revendications 9 à 14, où le médicament est pour l'utilisation dans le traitement d'un sujet humain en rémission temporaire de symptômes de la sclérose multiple.
